# EUROPEAN PATENT SPECIFICATION

(11) **EP 4 059 560 B1**
(45) Date of publication and mention of the grant of the patent: **05.11.2025**
(21) Application number: 22159739.6
(22) Date of filing: 02.03.2022
(51) Int. Cl.: A61N 1/02, A61N 1/36, G06F 13/00

(54) **DEVICE FOR TRANSMITTING DATA BETWEEN COILS AND DATA READING METHOD**
VORRICHTUNG ZUR DATENÜBERTRAGUNG ZWISCHEN SPULEN UND DATENLESEVERFAHREN
DISPOSITIF DE TRANSMISSION DE DONNÉES ENTRE DES BOBINES ET PROCÉDÉ DE LECTURE DE DONNÉES

(30) Priority: 19.03.2021 KR 20210036047
(43) Date of publication of application: 21.09.2022
(73) Proprietor: Todoc Co., Ltd., Seoul 08394 (KR)
(72) Inventor: PARK, Jong Hyeok, Daejeon 34138 (KR); LEE, Ho Seung, Seoul 05629 (KR); KIM, Doo Hee, Seoul 04075 (KR)
(74) Representative: Zimmermann, Tankred Klaus

(56) References cited:
- US-A1- 2017 118 543
- DAI JIANG ET AL: "An Integrated Passive Phase-Shift Keying Modulator for Biomedical Implants With Power Telemetry Over a Single Inductive Link", IEEE TRANSACTIONS ON BIOMEDICAL CIRCUITS AND SYSTEMS, vol. 11, no. 1, 1 February 2017 (2017-02-01), US, pages 64 - 77, XP055450207, ISSN: 1932-4545, DOI: 10.1109/TBCAS.2016.2580513
- QIAN XIN-HONG ET AL: "Design and In Vivo Verification of a CMOS Bone-Guided Cochlear Implant Microsystem", IEEE TRANSACTIONS ON BIOMEDICAL ENGINEERING, IEEE, USA, vol. 66, no. 11, 1 November 2019 (2019-11-01), pages 3156 - 3167, XP011750984, ISSN: 0018-9294, [retrieved on 20191017], DOI: 10.1109/TBME.2019.2901374
- SUSHMA P ET AL: "Implementation of Cochlear Implant Receiver Stimulator IC", 2017 14TH IEEE INDIA COUNCIL INTERNATIONAL CONFERENCE (INDICON), IEEE, 15 December 2017 (2017-12-15), pages 1 - 6, XP033418759, DOI: 10.1109/INDICON.2017.8487604

## Description

### BACKGROUND OF THE INVENTION

### Field of the Invention

The present disclosure relates to a device for transmitting data between coils and a data reading method. In particular, the present disclosure relates to a device for performing data transmission between coils provided in a cochlear implant and a data reading method of a reception end.

### Description of the Related Art

Document "An Integrated Passive Phase-Shift Keying Modulator for Biomedical Implants With Power Telemetry Over a Single Inductive Link" by Dai Jiang et al. (2017) discloses a passive phase-shift keying (PPSK) modulator for uplink data transmission for biomedical implants with simultaneous power and data transmission over a single 13.56 MHz inductive link.

Document US2017118543 A1 relates to a method and apparatus for efficiently transmitting power and data wirelessly over an inductive telemetry link, and more specifically to a modulation scheme for high bit rate, low BER data transfer with low power consumption.

Unless otherwise indicated herein, contents set forth in this section are neither the related art to the claims of the present application, nor construed as the related art despite the inclusion in this section.

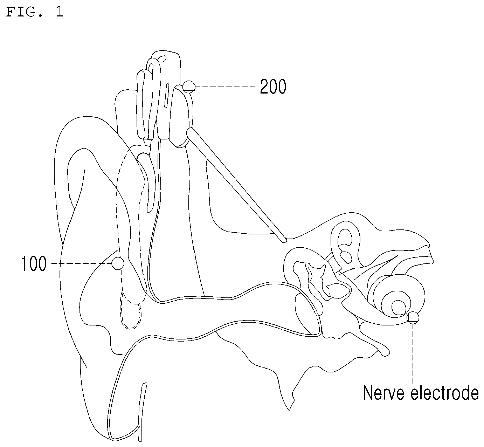

A cochlear implant is an electrical device that acts instead of damaged or lost hair cells and performs the functions of hair cells by giving electrical stimulation to the auditory nerve. The cochlear implant includes a speech processor and a nerve stimulator. FIG. 1 is a diagram illustrating a cochlear implant. A speech processor 100 of the cochlear implant receives sound, generates a stimulation pattern, transmits (TX) power and data to a nerve stimulator 200 of the cochlear implant, and receives the state of the nerve stimulator or nerve electrode from the nerve stimulator 200 to notify the user of the state (back-telemetry). The nerve stimulator 200 receives (RX) power and data from the speech processor 100, stimulates the auditory nerve through the nerve electrode by performing decoding, enables hearing to be regained, and transmits the nerve electrode state and a nerve reaction to the speech processor (back-telemetry). Herein, the conventional speech processor 100 and the conventional nerve stimulator 200 of the cochlear implant perform data transmission and reception therebetween through a back-telemetry method. Back-telemetry is a method of transmitting streaming data in reverse in a remote or wireless state so as to know the state of a device being monitored.

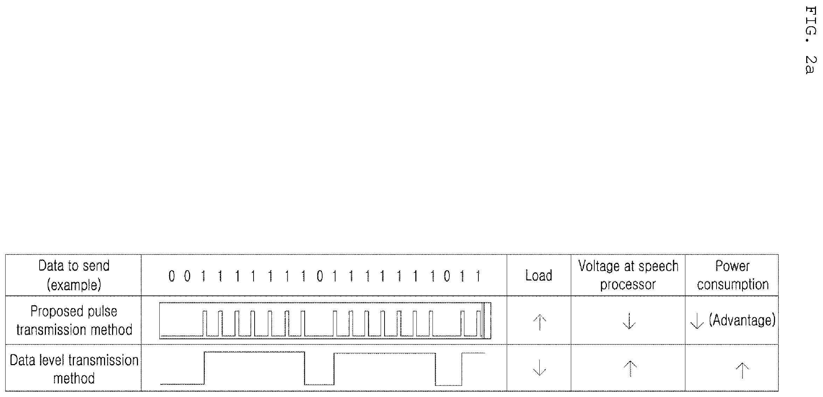

In order for the circuit of the nerve stimulator 200 to operate, power needs to be continuously supplied from the speech processor 100. However, it is impossible to supply power endlessly to the nerve stimulator 200 with the battery power set in the speech processor 100, so it is important to consume the power as efficiently as possible. In general, as a method (back-telemetry) of transmitting data from the circuit of the nerve stimulator 200 to the circuit of the speech processor 100, there is load-shift keying (LSK). This is a method of transmitting streaming data one bit by one bit while increasing or decreasing (shift) a relative equivalent model resistance (load) at the circuit of the nerve stimulator 200. For example, when the load is small, the voltage level at the speech processor circuit increases. When the load increases, the voltage level decreases. Conversely, the amount of power to be supplied to the nerve stimulator circuit increases when the load decreases. Therefore, data is transmitted in the form of a pulse rather than a level, so that the load is small and power consumption can be reduced. FIG. 2A is a table illustrating a comparison between data transmission using a pulse transmission method and a level transmission method in terms of a load, the voltage at a speech processor, and power consumption. As shown in FIG. 2A, it is found that the pulse transmission method has lower power consumption than the level transmission method.

In data transmission between the circuits of the cochlear implant in the related art, the nerve stimulator (RX end) transmits data to the speech processor (TX end) according to the mutually accepted transmission rate (bit per second), and whether the data is 1 or 0 is determined according to a result of comparing an input data level with a threshold voltage by a comparator circuit.

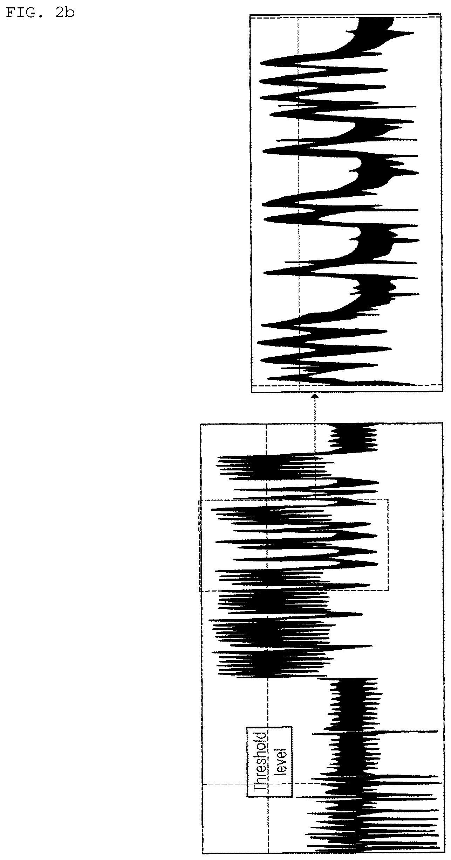

However, as shown in FIG. 2B, even if the nerve stimulator transmits data according to the ideal data rate, when the coil alignment between the TX and the RX is incorrect, a high-frequency noise signal is severely mixed with the original signal or the circuit of the speech processor 100 receives an input finally, with the momentarily low level. In this case, when the value of data is determined according to the accepted transmission rate, there is a frequently occurring problem that a value of 1 should be read, but a value of 0 is read at that moment.

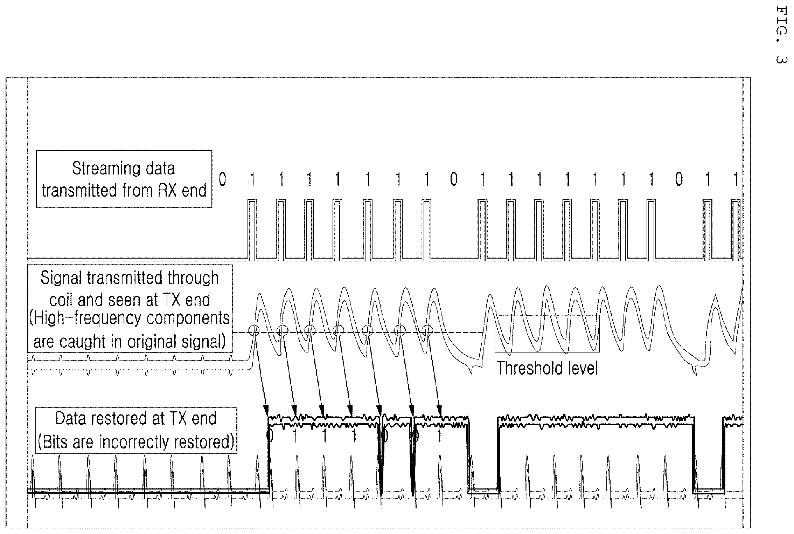

FIG. 3 is a diagram illustrating a case in which a problem occurred when the end of the speech processor restored an unrefined signal transmitted through coils in the related art. Referring to FIG. 3, because of a high-frequency noise signal or signal attenuation occurring when data is read at the time point that exactly matches the accepted transmission rate (bit per second), while decoding into a value of 1 is correct, incorrect decoding into a value of 0 may take place.

The foregoing is intended merely to aid in the understanding of the background of the present disclosure, and is not intended to mean that the present disclosure falls within the purview of the related art that is already known to those skilled in the art.

### Documents of Related Art

(Patent Document 1) Korean Patent Application Publication No. 10-2020-0007627 (22 January 2020)
(Patent Document 2) Korean Patent No. 10-0932204 (08 December 2009)

### SUMMARY OF THE INVENTION

In order to avoid the error that data is loaded according to a preset data rate cycle in a situation where a problem has occurred due to coil misalignment, a device for transmitting data between coils according to claim 1 and a data reading method according to claim 3 calculate a data reading timing margin on the basis of a fixed cycle that is a set system operation cycle, and read data at a time point resulting from adding the data reading timing margin to the fixed cycle.

According to an embodiment, there is provided a device for transmitting data between coils of a cochlear implant by using back-telemetry, the cochlear implant including: a speech processor (TX) configured to generate a stimulation pattern after receiving a sound and transmit power and data to a nerve stimulator; and the nerve stimulator (RX) configured to receive the power and the data from the speech processor, stimulate an auditory nerve through a nerve electrode by performing decoding, and transmit a nerve electrode state and nerve reaction data to the speech processor.

The nerve stimulator includes: a margin setting module configured to set a data reading timing margin on the basis of a system operation cycle that is a fixed cycle; and a data reading module configured to read the data at a time point after the set data reading timing margin from the fixed cycle.

According to another embodiment, there is provided a data reading method of a device for transmitting data between coils, wherein the device includes a transmission end (TX) and a reception end (RX), the method including: (A) setting, by the reception end, a data reading timing margin on the basis of a system operation cycle that is a fixed cycle; and (B) reading, by the reception end, data at a time point after the set data reading timing margin from the fixed cycle.

The device for transmitting data between coils and the data reading method described above acquire data according to a new cycle resulting from adding the data reading timing margin to the fixed cycle although an incorrect signal strength is output according to a predetermined data rate, thereby greatly reducing the possibility of an error of data determination by the reception end. In addition, the data reading method according to the embodiment simply calculates a new data acquisition cycle so that all the systems for transmitting and receiving streaming data between coils can acquire data without losing synchronization.

It should be understood that the effects are not limited to those described above, but include all effects that can be inferred from the configurations of the disclosure in the detailed description of the present disclosure or the claims.

### BRIEF DESCRIPTION OF THE DRAWINGS

The above and other objectives, features, and other advantages of the present disclosure will be more clearly understood from the following detailed description when taken in conjunction with the accompanying drawings, in which:
FIG. 1 is a diagram illustrating a cochlear implant;
FIG. 2A is a table illustrating a comparison between data transmission using a pulse transmission method and a level transmission method in terms of a load, the voltage at a speech processor, and power consumption;
FIG. 2B is a diagram illustrating the form of an unrefined signal at the speech processor which was transmitted through the coil;
FIG. 3 is a diagram illustrating a case in which a problem occurred when the end of the speech processor restored an unrefined signal transmitted through the coil;
FIG. 4 is a diagram illustrating a configuration of a cochlear implant that is a device for transmitting data between coils according to an embodiment;
FIG. 5 is a diagram illustrating a data processing configuration of a nerve stimulator according to an embodiment;
FIG. 6 is a diagram illustrating a data processing configuration of a data reading module 230 according to an embodiment;
FIGS. 7A and 7B are diagrams illustrating a received-data processing and data restoration process of a device for transmitting data between coils according to an embodiment; and
FIG. 8 is a flowchart illustrating a data reading method of a device for transmitting data according to an embodiment.

### DETAILED DESCRIPTION OF THE INVENTION

Advantages and features of the present disclosure, and methods to achieve them will be apparent from the following embodiments that will be described in detail with reference to the accompanying drawings. It should be understood that the present disclosure is not limited to the following embodiments and may be embodied in different ways, and that the embodiments are provided to only complete the disclosure and to allow those skilled in the art to fully understand the category of the disclosure. Throughout the specification, the same reference numerals refer to the same elements.

In describing the embodiments of the present disclosure, when if it is decided that a detailed description of a well-known function or configuration makes the gist of the present disclosure unclear, the detailed description will be omitted. Further, the terms described below are defined in consideration of the functions in the embodiments of the present disclosure, and may vary depending on the intention of the user, the operator, or the custom. Therefore, the definition should be based on the contents throughout this specification.

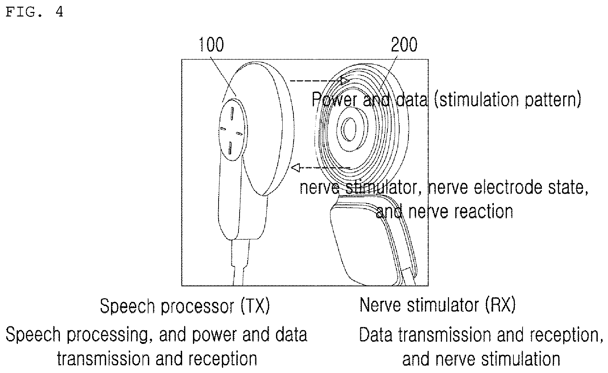

FIG. 4 is a diagram illustrating a configuration of a cochlear implant that is a device for transmitting data between coils according to an embodiment.

Referring to FIG. 4, the cochlear implant may include a speech processor 100 and a nerve stimulator 200. As a transmission end, the speech processor 100 receives a sound from the outside, generates a stimulation pattern, and transmits (TX) power and data to the nerve stimulator 200. In addition, as a reception end, the speech processor 100 receives nerve stimulator or nerve electrode state data from the nerve stimulator 200 and notifies the user of this (back-telemetry).

The nerve stimulator 200 receives (RX) power and data from the speech processor 100, and performs decoding to stimulate the auditory nerve through a nerve electrode, and enables hearing to be regained. The nerve stimulator 200 transmits nerve electrode state and nerve reaction data to the speech processor 100 (back-telemetry). The nerve stimulator 200 and the speech processor 100 according to the embodiment perform data transmission and reception between the nerve stimulator and the speech processor through back-telemetry. Back-telemetry is transmitting streaming data in reverse in a remote or wireless state so as to know the state of a device being monitored.

In a cochlear implant system according to an embodiment, back-telemetry means transmitting data to the speech processor (TX end) 100 through coils so as to monitor the state of the nerve stimulator (RX end) 200. In addition, in an embodiment, through the back-telemetry method, the speech processor 100 may receive nerve stimulator or nerve electrode state information from the nerve stimulator 200 and may notify the user of the same.

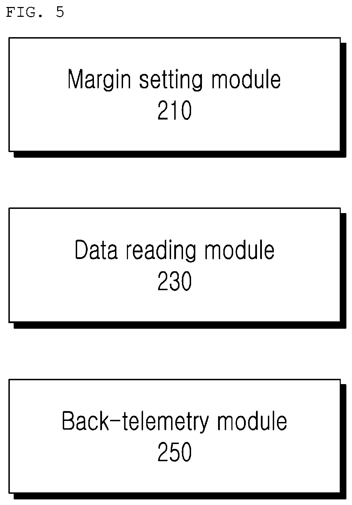

FIG. 5 is a diagram illustrating a data processing configuration of a nerve stimulator according to an embodiment.

Referring to FIG. 5, the nerve stimulator according to the embodiment may include a margin setting module 210, a data reading module 230, and a back-telemetry module 250. The term "module" used herein should be interpreted to include software, hardware, or a combination thereof, depending on the context in which the term is used. For example, software may be machine language, firmware, embedded code, and application software. As another example, hardware may be a circuit, a processor, a computer, an integrated circuit, an integrated circuit core, a sensor, a micro-electro-mechanical system (MEMS), a passive device, or a combination thereof.

The margin setting module 210 sets a data reading timing margin on the basis of a system operation cycle that is a fixed cycle. For example, the margin setting module 210 may set a value corresponding to a predetermined ratio of a set data decoding rate cycle as a margin. Specifically, when the data decoding rate cycle is set such that the value of data is decoded one bit by one bit every 10 cycles, the margin setting module 210 sets 2 cycles, which is 20 % of the set cycle, as the data reading timing margin of 10 cycles, and sets 12 cycles as a data reception cycle. That is, the margin setting module 210 according to the embodiment may set the reception cycle of 12 cycles in preparation for the attenuation of the signal strength due to coil change factors.

When the data transmitted from the nerve stimulator has a value of 0, the value is lower than a comparator threshold level of the speech processor and determined as 0 without any problem. However, when the data transmitted from the nerve stimulator has a value of 1, there may be a problem that at the timing of the 10th cycle, the threshold voltage is not exceeded because of a noise. Therefore, in the embodiment, data is restored after monitoring whether the signal increases above the threshold level for up to 12 cycles including the margin cycle.

The data reading module 230 reads data at the time point after the reading timing margin from the fixed cycle. In the embodiment, the data reading module 230 reads data at the time point after the data reading timing margin of several clocks (cycles) from the operation clocks, so that even an incorrect signal strength is obtained in the fixed cycle, the data obtained in a new cycle resulting from adding the data reading timing margin is used for decoding. Accordingly, the error of data determination by the reception end can be greatly reduced.

The back-telemetry module 250 transmits the nerve electrode state and the nerve reaction data read by the data reading module 230 to the speech processor.

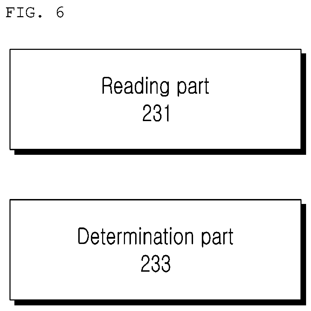

FIG. 6 is a diagram illustrating a data processing configuration of a data reading module 230 according to an embodiment.

Referring to FIG. 6, the data reading module 230 according to the embodiment may include a reading part 231 and a determination part 233. The reading part 231 reads data at the time point after the reading timing margin from the fixed cycle when the data transmitted to the nerve stimulator has a value of 0. The determination part 233 compares the data obtained at the time point after the reading timing margin with a threshold voltage level, and determines the data according to a result of comparison.

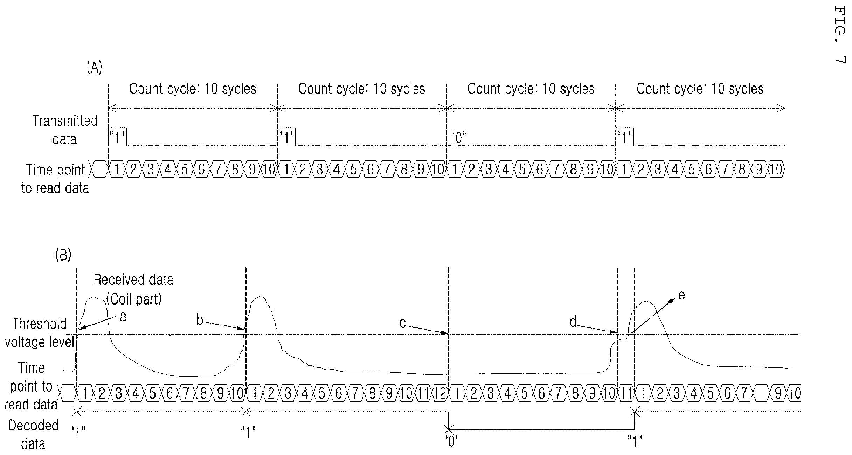

FIGS. 7A and 7B are diagrams illustrating a received-data processing and data restoration process of a device for transmitting data between coils according to an embodiment.

FIG. 7A is a diagram illustrating cycles in which data is transmitted from the nerve stimulator (RX end) to the speech processor (TX end). Referring to FIG. 7A, it is found that data was transmitted from the nerve stimulator to the speech processor according to the fixed cycle of 10 cycles.

FIG. 7B is a diagram illustrating a process of reading coil received data and restored data by the speech processor (TX end). Referring to FIG. 7B, the device for transmitting data between coils according to the embodiment loads a data value in the 12th cycle resulting from adding the data reading timing margin of 2 cycles to the fixed cycle of 10 cycles. Specifically, at time point a and time point b, the received data read in the 10th cycle among the 10 cycles exceeds the threshold voltage and is decoded to 1. At time point c, the data value changes from 1 to 0 in the 10th cycle, so waiting for 2 cycles that is the data reading timing margin cycle takes place and the received data is read as 0 in the 12th cycle. At time point d, although the intended value of 1 is not obtained in the 10th cycle, a correct value of 1 is read at time point e that is the 11th cycle owing to the data reading timing margin.

In the embodiment, when the bit having a value of 1 is loaded as the received data, the value of the fixed cycle that is the set system operation cycle is loaded and the count is initialized. When the value is not loaded until the cycle (for example, 12th cycle) resulting from adding the margin, the bit having a value of 0 is loaded and the count is initialized.

Hereinafter, a data reading method of the device for transmitting data between coils will be described in order. The operation (function) of the data reading method according to the embodiment is fundamentally the same as the function of the device for transmitting data between coils, so a description the same as that of FIGS. 1 to 7B will be omitted.

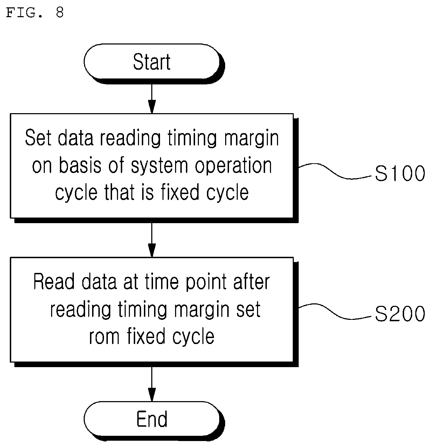

FIG. 8 is a flowchart illustrating a data reading method of the device for transmitting data according to an embodiment.

When the transmission end of the device for transmitting data between coils transmits data to the reception end, the data reception end sets a data reading timing margin on the basis of a system operation cycle that is a fixed cycle at step S100. In the embodiment, the data reading timing margin may be set as a predetermined ratio of the fixed cycle. Afterward, the reception end reads data at the time point after the set reading timing margin from the fixed cycle at step S200. In the embodiment, at step S200, when the received bit has a value of 0, data is read at the time point after the reading timing margin (cycle) from the fixed cycle and the data obtained at the time point after the reading timing margin is compared with the preset threshold voltage level. Afterward, received data is determined according to a result of comparison. That is, at step S200, data is obtained according to a new cycle resulting from adding the reading timing margin to the fixed cycle.

For example, when the fixed cycle set in the system is 10 cycles and the value of data is decoded one bit by one bit every 10 cycles, the data reading timing margin is added to set the reception cycle of 12 cycles in preparation for the attenuation of the signal strength due to coil change factors. When the received data has a value of 0, the value is lower than the comparator threshold level and there is no problem in data determination. When the received data has a value of 1, a problem may occur at the timing of the 10th cycle. Therefore, in the embodiment, data is determined after monitoring whether the signal increases above the threshold level for up to 12 cycles. In the embodiment, by providing 2 cycles as the data reading timing margin in addition to 10 cycles, correct data can be received even when the timing changes up to 20 %. In addition, this method enables all the systems for transmitting and receiving streaming data through coils to obtain correct data simply without losing synchronization.

At step S200, when the bit received from the transmission end has a value of 1, the data is read as having a value of 1 and the count is initialized. When the data is not loaded from the speech processor until after the reading timing margin, the bit having a value of 0 is loaded and the count is initialized.

In the device for transmitting data between coils and the data reading method according to the embodiment of the present disclosure, if the received data is read within the reading timing margin because of a noise or signal attenuation when the received data having a value of 0 continues or the received data has a value of 1, the reading timing margin may be accumulated.

The data protocol to which the embodiment of the present disclosure is applied may be defined as transmitting and receiving a value of 0 twice or less. According to the present disclosure, although the data reading timing is the 10th cycle, when the received data exceeds the threshold voltage level before the 10th cycle, the data is read as having a value of 1 According to this embodiment of the present disclosure, there is no risk of loss of received data even though the data reading timing is delayed for 1 to 2 cycles.

The device for transmitting data between coils and the data reading method described above acquire data according to a new cycle resulting from adding the data reading timing margin to the fixed cycle although an incorrect signal strength is output according to a predetermined data rate, thereby greatly reducing the possibility of an error of data determination by the reception end. In addition, the data reading method according to the embodiment simply calculates a new data acquisition cycle so that all the systems for transmitting and receiving streaming data between coils can acquire data without losing synchronization.

Although preferred embodiments of the present disclosure have been described for illustrative purposes, those skilled in the art will appreciate that various modifications, additions, and substitutions are possible, without departing from the scope of the disclosure. The scope of the invention is defined in the accompanying claims.

## Claims

1. A device for transmitting data between coils by using back-telemetry, the device comprising:
a speech processor (100), TX, configured to generate a stimulation pattern after receiving a sound and transmit power and data to a nerve stimulator (200); and
the nerve stimulator (200), RX, configured to receive the power and the data from the speech processor (100), stimulate an auditory nerve through a nerve electrode by decoding the data received from the speech processor (100), and transmit a nerve electrode state and nerve reaction data to the speech processor (100),
wherein the nerve stimulator (200) comprises:
a margin setting module (210) configured to set a data reading timing margin on the basis of a system operation cycle that is a fixed cycle; and
a data reading module (230) configured to read the data at a time point after the set data reading timing margin from the fixed cycle,
**characterised in that** the data reading module (230) comprises:
a reading part (231) configured to read the data at the time point after the data reading timing margin from the fixed cycle when a received bit has a value of 0; and
a determination part (233) configured to compare the data obtained at the time point after the data reading timing margin with a preset threshold voltage level, wherein the obtained data is determined to have a bit value of 1 if the obtained data is higher than the preset threshold voltage level and to have a bit value of 0 if the obtained data is lower than the preset threshold voltage level,
wherein the data reading module (230) is configured to read the data as having a value of 1 and initialize a count when the bit received from the speech processor (100) has a value of 1,
wherein the data reading module (230) is configured to load a bit having a value of 0 and initialize a count when the data is not loaded from the speech processor (100) until after the data reading timing margin,
wherein the margin setting module (210) is configured to set 20 % of the fixed cycle as the data reading timing margin, and
wherein a data protocol applied to the data reading module (230) is limited to transmitting and receiving a value of 0 twice or less.

2. The device of claim 1, wherein the nerve stimulator (200) further comprises a back-telemetry module (250) configured to transmit the nerve electrode state and the nerve reaction data to the speech processor (100).

3. A data reading method of a device for transmitting data between coils, wherein the device includes a transmission end, TX, and a reception end, RX, wherein the method comprises:
(A) setting, by the reception end, a data reading timing margin on the basis of a system operation cycle that is a fixed cycle; and
(B) reading, by the reception end, data at a time point after the set data reading timing margin from the fixed cycle,
**characterised in that** the step (B) comprises:
(B-1) reading the data at the time point after the data reading timing margin from the fixed cycle when a received bit has a value of 0; and
(B-2) comparing the data obtained at the time point after the data reading timing margin with a preset threshold voltage level, wherein the received data is determined to have a bit value of 1 if the obtained data is higher than the preset threshold voltage level and to have a bit value of 0 if the obtained data is lower than the preset threshold voltage,
wherein at the step (B-2), when the bit received from the transmission end has a value of 1, the data is read as having a value of 1 and a count is initialized,
wherein at the step (B-2), when the data is not loaded from the speech (100) until after the data reading timing margin, a bit having a value of 0 is loaded and a count is initialized,
wherein setting the data reading timing margin comprises setting 20 % of the fixed cycle as the data reading timing margin, and
wherein a data protocol applied to reading the data is limited to transmitting and receiving a value of 0 twice or less.

## Patentansprüche

1. Eine Vorrichtung zum Senden von Daten zwischen Spulen unter Verwendung von Rücktelemetrie, wobei die Vorrichtung folgende Merkmale aufweist:
einen Sprachprozessor (100), TX, der dazu konfiguriert ist, ein Stimulationsmuster nach Empfangen eines Tons zu erzeugen und Leistung und Daten an einen Nervenstimulator (200) zu senden; und
den Nervenstimulator (200), RX, der dazu konfiguriert ist, die Leistung und die Daten von dem Sprachprozessor (100) zu empfangen, einen Hörnerv über eine Nervenelektrode durch Decodieren der Daten, die von dem Sprachprozessor (100) empfangen werden, zu stimulieren, und einen Nervenelektrodenzustand und Nervenreaktionsdaten an den Sprachprozessor (100) zu senden,
wobei der Nervenstimulator (200) folgende Merkmale aufweist:
ein Spanneneinstellungsmodul (210), das dazu konfiguriert ist, eine Datenlesezeitspanne basierend auf einem Systemoperationszyklus einzustellen, der ein fester Zyklus ist; und
ein Datenlesemodul (230), das dazu konfiguriert ist, die Daten zu einem Zeitpunkt nach der eingestellten Datenlesezeitspanne aus dem festen Zyklus zu lesen,
**dadurch gekennzeichnet, dass** das Datenlesemodul (230) folgende Merkmale aufweist:
einen Leseabschnitt (231), der dazu konfiguriert ist, die Daten zu dem Zeitpunkt nach der Datenlesezeitspanne aus dem festen Zyklus zu lesen, wenn ein empfangenes Bit einen Wert von 0 aufweist; und
einen Bestimmungsabschnitt (233), der dazu konfiguriert ist, die Daten, die zu dem Zeitpunkt nach der Datenlesezeitspanne erhalten werden, mit einem voreingestellten Schwellenspannungspegel zu vergleichen, wobei bestimmt wird, dass die erhaltenen Daten einen Bitwert von 1 aufweisen, wenn die erhaltenen Daten höher als der voreingestellte Schwellenspannungspegel sind, und einen Bitwert von 0 aufweisen, wenn die erhaltenen Daten niedriger als der voreingestellte Schwellenspannungspegel sind,
wobei das Datenlesemodul (230) dazu konfiguriert ist, die Daten derart zu lesen, dass dieselben einen Wert von 1 aufweisen, und eine Zählung zu initialisieren, wenn das Bit, das von dem Sprachprozessor (100) empfangen wird, einen Wert von 1 aufweist,
wobei das Datenlesemodul (230) dazu konfiguriert ist, ein Bit mit einem Wert von 0 zu laden und eine Zählung zu initialisieren, wenn die Daten erst nach der Datenlesezeitspanne aus dem Sprachprozessor (100) geladen werden,
wobei das Grenzwerteinstellungsmodul (210) dazu konfiguriert ist, 20 % des festen Zyklus als die Datenlesezeitspanne einzustellen, und
wobei ein Datenprotokoll, das auf das Datenlesemodul (230) angewendet wird, darauf beschränkt ist, einen Wert von 0 zweimal oder weniger zu senden und zu empfangen.

2. Die Vorrichtung gemäß Anspruch 1, wobei der Nervenstimulator (200) ferner ein Rücktelemetriemodul (250) aufweist, das dazu konfiguriert ist, den Nervenelektrodenzustand und die Nervenreaktionsdaten an den Sprachprozessor (100) zu senden.

3. Ein Datenleseverfahren einer Vorrichtung zum Senden von Daten zwischen Spulen, wobei die Vorrichtung ein Sendungsende, TX, und ein Empfangsende, RX, umfasst, wobei das Verfahren folgende Schritte aufweist:
(A) Einstellen, durch das Empfangsende, einer Datenlesezeitspanne basierend auf einem Systemoperationszyklus, der ein fester Zyklus ist; und
(B) Lesen, durch das Empfangsende, von Daten zu einem Zeitpunkt nach der eingestellten Datenlesezeitspanne aus dem festen Zyklus,
**dadurch gekennzeichnet, dass** der Schritt (B) folgende Schritte aufweist:
(B-1) Lesen der Daten zu dem Zeitpunkt nach der Datenlesezeitspanne aus dem festen Zyklus, wenn ein empfangenes Bit einen Wert von 0 aufweist; und
(B-2) Vergleichen der Daten, die zu dem Zeitpunkt nach der Datenlesezeitspanne erhalten werden, mit einem voreingestellten Schwellenspannungspegel, wobei bestimmt wird, dass die empfangenen Daten einen Bitwert von 1 aufweisen, wenn die erhaltenen Daten höher als der voreingestellte Schwellenspannungspegel sind, und einen Bitwert von 0 aufweisen, wenn die erhaltenen Daten niedriger als der voreingestellte Schwellenspannungspegel sind,
wobei bei dem Schritt (B-2) dann, wenn das Bit, das aus dem Sendungsende empfangen wird, einen Wert von 1 aufweist, die Daten derart gelesen werden, dass dieselben einen Wert von 1 aufweisen, und eine Zählung initialisiert wird,
wobei bei dem Schritt (B-2) dann, wenn die Daten erst nach der Datenlesezeitspanne aus dem Sprachprozessor (100) geladen werden, ein Bit mit einem Wert von 0 geladen wird und eine Zählung initialisiert wird,
wobei das Einstellen der Datenlesezeitspanne ein Einstellen von 20 % des festen Zyklus als die Datenlesezeitspanne aufweist, und
wobei ein Datenprotokoll, das auf das Lesen der Daten angewendet wird, darauf beschränkt ist, einen Wert von 0 zweimal oder weniger zu senden und zu empfangen.

## Revendications

1. Dispositif de transmission de données entre des bobines en utilisant la rétro-télémétrie, le dispositif comprenant :
un processeur de parole (100), TX, configuré pour générer un motif de stimulation après réception d'un son et transmettre de la puissance et des données à un neurostimulateur (200) ; et
le neurostimulateur (200), RX, étant configuré pour recevoir la puissance et les données provenant du processeur de parole (100), stimuler un nerf auditif par le biais d'une électrode de nerf en décodant les données reçues du processeur de parole (100), et transmettre un état d'électrode de nerf et des données de réaction de nerf au processeur de parole (100),
dans lequel le neurostimulateur (200) comprend :
un module de réglage de marge (210) configuré pour régler une marge de temporisation de lecture de données sur la base d'un cycle de fonctionnement de système qui est un cycle fixe ; et
un module de lecture de données (230) configuré pour lire les données à un moment après la marge de temporisation de lecture de données réglée depuis le cycle fixe,
**caractérisé en ce que** le module de lecture de données (230) comprend :
une partie de lecture (231) configurée pour lire les données au moment après la marge de temporisation de lecture de données depuis le cycle fixe lorsqu'un bit reçu présente une valeur de 0 ; et
une partie de détermination (233) configurée pour comparer les données obtenues au moment après la marge de temporisation de lecture de données à un niveau de tension seuil prédéfini, dans lequel les données obtenues sont déterminées pour présenter une valeur de bit de 1 si les données obtenues sont supérieures au niveau de tension seuil prédéfini et pour présenter une valeur de bit de 0 si les données obtenues sont inférieures au niveau de tension seuil prédéfini,
dans lequel le module de lecture de données (230) est configuré pour lire les données comme présentant une valeur de 1 et initialiser un compte lorsque le bit reçu du processeur de parole (100) présente une valeur de 1,
dans lequel le module de lecture de données (230) est configuré pour charger un bit présentant une valeur de 0 et initialiser un compte lorsque les données ne sont pas chargées depuis le processeur de parole (100) jusqu'à après la marge de temporisation de lecture de données,
dans lequel le module de réglage de marge (210) est configuré pour régler 20 % du cycle fixe en tant que marge de temporisation de lecture de données, et
dans lequel un protocole de donnés appliqué au module de lecture de données (230) est limité à la transmission et à la réception d'une valeur de 0 deux fois ou moins.

2. Dispositif selon la revendication 1, dans lequel le neurostimulateur (200) comprend en outre un module de rétro-télémétrie (250) configuré pour transmettre l'état d'électrode de nerf et les données de réaction de nerf au processeur de parole (100).

3. Procédé de lecture de données d'un dispositif de transmission de données entre des bobines, dans lequel le dispositif inclut une extrémité de transmission, TX, et une extrémité de réception, RX, dans lequel le procédé comprend le fait de :
(A) régler, par l'extrémité de réception, une marge de temporisation de lecture de données sur la base d'un cycle de fonctionnement de système qui est un cycle fixe ; et
(B) lire, par l'extrémité de réception, des données à un moment après la marge de temporisation de lecture de données réglée depuis le cycle fixe, **caractérisé en ce que** l'étape (B) comprend le fait de :
(B-1) lire les données au moment après la marge de temporisation de lecture de données depuis le cycle fixe lorsqu'un bit reçu présente une valeur de 0 ; et
(B-2) comparer les données obtenues au moment après la marge de temporisation de lecture de données à un niveau de tension seuil prédéfini, dans lequel les données reçues sont déterminées pour avoir une valeur de bit de 1 si les données obtenues sont supérieures au niveau de tension seuil prédéfini et pour présenter une valeur de bit de 0 si les données obtenues sont inférieures au niveau de tension seuil prédéfini,
dans lequel, à l'étape (B-2), lorsque le bit reçu de l'extrémité de transmission présente une valeur de 1, les données sont lues comme présentant une valeur de 1 et un compte est initialisé,
dans lequel, à l'étape (B-2), lorsque les données ne sont pas chargées à partir de la parole (100) jusqu'à après la marge de temporisation de lecture de données, un bit présentant une valeur de 0 est chargé et un compte est initialisé,
dans lequel le réglage de la marge de temporisation de lecture de données comprend le réglage de 20 % du cycle fixe en tant que marge de temporisation de lecture de données, et
dans lequel un protocole de données appliqué à la lecture des données est limité à la transmission et à la réception d'une valeur de 0 deux fois ou moins.
